Europäisches Patentamt

(19) European Patent Office

Office européen des brevets

(11) Publication number : **0 516 704 B1**

(12) # EUROPEAN PATENT SPECIFICATION

(45) Date of publication of patent specification :
**10.11.93 Bulletin 93/45**

(51) Int. Cl.⁵ : **A61K 37/26, // C07K7/42**

(21) Application number : **91904980.9**

(22) Date of filing : **21.02.91**

(86) International application number :
**PCT/DK91/00052**

(87) International publication number :
**WO 91/12817 05.09.91 Gazette 91/21**

(54) **NOVEL INSULIN COMPOSITIONS.**

The file contains technical information
submitted after the application was filed and
not included in this specification

(30) Priority : **21.02.90 DK 455/90**

(43) Date of publication of application :
**09.12.92 Bulletin 92/50**

(45) Publication of the grant of the patent :
**10.11.93 Bulletin 93/45**

(84) Designated Contracting States :
**AT BE CH DE DK ES FR GB GR IT LI LU NL SE**

(56) References cited :
**DE-A- 971 500**
**DE-A- 1 107 374**

(56) References cited :
**Chemical Abstracts, vol. 102, no. 19, 13 May**
**1985, Columbus, Ohio, US, Storm, M. C et al., p.**
**61, abstract no. 160544h**
**Chemical Abstracts, vol. 109, no. 5, 1 August**
**1988, Columbus, Ohio, US, Roy, M. et al., p. 79,**
**abstract no. 32217m**

(73) Proprietor : **NOVO NORDISK A/S**
**Novo Allé**
**DK-2880 Bagsvaerd (DK)**

(72) Inventor : **HANSEN, Jorgen, Frederik**
**Ordrupvej 23, 2.t.v.**
**DK-2920 Charlottenlund (DK)**
Inventor : **RIBEL-MADSEN, Ulla**
**Birkely 1**
**DK-2830 Virum (DK)**

(74) Representative : **Jorgensen, Dan et al**
**Novo Nordisk A/S, Patent Department, Novo**
**Allé**
**DK-2880 Bagsvaerd (DK)**

EP 0 516 704 B1

EP 0 516 704 B1

**Description**

The present invention relates to protracted insulin compositions soluble at physiological pH and containing insulin complexes of Co(III) and to the use of such insulin complexes for the manufacture of pharmaceutical compositions for the treatment of diabetes mellitus.

Many diabetic patients are treated with multiple daily insulin injections in a regimen comprising one or two daily injections of a protracted insulin to cover the basal needs supplemented by bolus injections of a rapid acting insulin to cover the requirement related to meals.

Protracted insulin compositions are well known in the art. Thus one main type of protracted insulin compositions comprises injectable aqueous suspensions of insulin crystals or amorphous insulin. In these compositions the insulin compounds utilized typically are protamine insulin, zinc insulin or protamine zinc insulin.

Certain drawbacks are associated with the use of insulin suspensions. Thus in order to secure an accurate dosing the insulin particles must be suspended homogeneously by gentle shaking before a defined volume is withdrawn from a vial or expelled from a cartridge. Also for the storage of insulin suspensions the temperature must be kept within more narrow limits than for insulin solutions in order to avoid lump formation or coagulation.

While it was earlier believed that protamines were non-immunogenic it has now turned out that protamines can be immunogenic in man and that their use for medical purposes may lead to formation of antibodies (Samuel, T. et al., Studies on the immunogenecity of protamines in humans and experimental animals by means of a micro-complement fixation test. Clin. Exp. Immunol. 33 (1978)252-260).

Also evidence has been found that the protamine-insulin complex is itself immunogenic (Kurtz, A.B. et al., Circulating IgG antibody to protamine in patients treated with protamine-insulins. Diabetologica 25 (1983)322-324). Therefore, with some patients the use of protracted insulin compositions containing protamines must be avoided.

Another type of protracted insulin compositions are solutions having a pH below physiological pH from which the insulin will precipitate because of the rise in pH when the solution is injected.

A drawback with these solutions is that the particle size distribution of the precipitate formed in the tissue on injection and thus the timing of the medication depends on the blood flow at the injection site and other parameters in a somewhat unpredictable manner.

A further drawback is that the solid particles of the insulin may act as a local irritant causing inflammation of the tissue of the injection site.

Administration of a prior art insulin composition in a relatively high dose may under adverse conditions lead to hypoglycaemia, for example if the absorption from the injection site occurs more rapidly than intended. Hypoglycaemia may set in rather suddenly and hypoglycaemic patients need immediate treatment as hypoglycaemia if untreated can be fatal.

Accordingly, there is a need for protracted injectable insulin compositions which are solutions and contain insulins which stay in solution after injection and possess minimal inflammatory and immunogenic properties and which have a reduced tendency to cause hypoglycaemia.

The present invention is based on the surprising fact that certain insulin complexes of cobalt in the oxidation state +3, also - according to the Stock nomenclature - designated as Co(III), have a protracted insulin effect in vivo. The complexes are soluble at physiological pH. Thus injection of their solutions does not lead to the formation of inflammatory solid particles in the tissue at the injection site. When compositions according to the invention was administered to rabbits in doses which would normally have lead to hypoglycaemia no sign of hypoglycaemia was observed.

Within the context of this invention the term insulin when used in a plural or generic sense is intended to encompass both naturally occuring insulins and insulin analogues.

In its broadest aspect the present invention provides novel protracted insulin compositions which comprise an insulin complex of the general formula I

$$(Ins)_6(Co(III))_2(L)_m(M)_n \qquad (I)$$

wherein Ins is a naturally occuring insulin or an insulin analogue which has insulin effect in humans, L is a nitrogen containing ligand which can bond to Co(III) via a nitrogen atom, M is a ligand comprising no nitrogen atom such as a water molecule or an anion which can bond to Co(III) with the proviso that if n is 2 or higher, M may designate different species at the same time and n is zero or an integer between 1 and 6, and m is 6-n, when L is a monodentate ligand or (6 - n)/2, when L is a bedentate ligand and a suitable carrier for use as a medicament.

A first group of preferred compositions according to this invention contains insulin complexes of the general formula I wherein m is zero.

Another group of preferred compositions according to this invention contains insulin complexes of the general formula I wherein M is water.

2

A further group of preferred compositions according to this invention contains insulin complexes of the general formula I wherein Ins is bovine insulin.

A further group of preferred compositions according to this invention contains insulin complexes of the general formula I wherein Ins is porcine insulin.

A further group of preferred compositions according to this invention contains insulin complexes of the general formula I wherein Ins is human insulin.

A further group of preferred compositions according to this invention contains insulin complexes of the general formula I in a concentration of between 10 IU and 2000 IU, preferably between 40 IU and 200 IU per ml.

Ligands coordinate to Co(III) with widely varying affinity and it is thus contemplated that this variation can be utilized to produce compositions with a tailored release profile.

In the general formula I, L may for example be ammonia, TRIS, methylamine, ethylamine, propylamine, ethylenediamine, 1,2-diaminopropane, 1,3-diaminopropane, imidazole or histidine.

In the general formula I, M may for example be water, $OH^-$, $Cl^-$, $Br^-$, $I^-$, $SO_4^{2-}$, $HSO_4^-$, $PO_4^{3-}$, $HPO_4^{2-}$ or $H_2PO_4^-$, preferably $H_2O$, $OH^-$ or $Cl^-$.

The compositions according to this invention are useful for the treatment of diabetes mellitus.

The pursuit of new insulins and insulin compositions with improved properties has taken place since insulin was first discovered, and basic screening of potential new insulins is usually performed in in vitro models.

It seems evident that a necessary condition that a drug can exert its activity is that it binds to its receptor. Therefore, in one basic screening model the binding of potential new insulins to the insulin receptor is investigated in vitro. Until now, it has been found that potential new insulins with a good insulin activity in pigs, dogs, or humans exhibit a good receptor binding even in vitro.

Another in vitro model which can be used to screen for insulin activity is the widely recognized free fat cell bioassay (Moody, A.J. et al., A simple Free Fat Cell Bioassay for Insulin. Horm. Metab. Res. 6 (1974) 12-16). In this assay the efficiency of the test compound to mediate the incorporation of glucose into the lipids of isolated rat fat cells is taken as a measure of its insulin activity.

Also with this model experience has shown, that a favourable insulin activity in pigs, dogs, or humans can only be expected from test compounds which perform well in this test, i.e. are potent mediators of the incorporation of glucose into lipids.

The preparation of a Co(III) complex of bovine insulin of the formula II:

$$(Ins)_6(Co(III))_2 \qquad (II)$$

wherein Ins is bovine insulin has been described (Storm, M.C. et al., The Glu(B13) Carboxylates of the Insulin Hexamer Form a Cage for $Cd^{2+}$ and $Ca^{2+}$ Ions. Biochemistry 24 (1985) 1749-1756). The complex was obtained by first forming a Co(II) complex with metal-free bovine insulin and subsequently oxidizing the product formed with hydrogen peroxide. The final product is water-soluble, and the solution has a pink colour. Heretofore no mention has been made of any insulin activity of the complex.

When tested in the receptor-binding model it turned out that the complex of formula (II) did not bind to the insulin receptor.

Also when tested in the free fat cell assay the complex of formula (II) turned out to be devoid of measurable insulin activity.

Surprisingly, however, it has now turned out that when a composition comprising the complex of formula (II) is tested in vivo by injection into pigs and rabbits it exhibits 50-100 per cent insulin activity with a protracted insulin profile.

Animal experiments indicate that high dose levels of $Ins_6(Co(III))_2$ compared to conventional insulin compositions do not give life-threatening hypoglycaemia. Thus a more safe treatment of diabetes is possible with the compositions of the invention. Being solutions the compositions of the invention have a more reproducible absorption than compositions containing insulin crystals or amorphous insulin.

The injectable insulin compositions of the invention can be prepared following the conventional techniques of the pharmaceutical industry involving dissolving and mixing the ingredients as appropriate to give the desired end product.

Thus according to one procedure the insulin complex of formula I can be dissolved in an amount of water which is somewhat less than the final volume of the composition to be prepared. Following the possible addition of an isotonic agent, a preservative and possibly a buffer, an acid, or a base the volume of the solution can be adjusted with water to give the desired concentration of the ingredients.

Examples of preservatives are phenol, cresol, methyl p-hydroxybenzoate and benzyl alcohol.

Examples of suitable buffers are sodium acetate and sodium phosphate.

Preferably, none of the auxiliary compounds are strong reducing agents.

The insulin compositions of this invention can be used in the treatment of diabetes. It is recommended that the dosage of the insulin compositions of this invention be determined by a physician in a similar way as for known insulin compositions.

The features disclosed in the above specification and the following examples and claims may, both separately and in any combination thereof, be material for realizing this invention in diverse forms thereof.

The following examples are not to be construed as limiting but merely as an illustration of some preferred embodiments of the invention.

Example 1

Preparation of $(Inh)_6(Co(III))_2$ with water coordinated

To 4 ml of metal-free human insulin (Inh) (100 mg/ml) was added 3 ml of 0.01 M cobaltous chloride. pH was adjusted to 7.5 by means of 0.1 N sodium hydroxide and the mixture was allowed to stand for 10 minutes. 30% hydrogen peroxide (70 $\mu$l) was added to the colourless solution which developed a pink colour. The mixture was stirred for 1 hr. whereupon unreacted insulin was separated by size chromatography on Sephadex® G 100. Buffer: 0.02 M Tris plus 0.001 M EDTA. The $(Inh)_6(Co(III))_2$-containing fractions were pooled, dialyzed, and concentrated by ultrafiltration. Finally, the ultrafiltrate was lyophilized.

Example 2

Preparation of $(Inh)_6(Co(III))_2$ with water coordinated

To a solution of 55 mg (9.5 $\mu$mole) of metal-free human insulin in 5 ml of water was added 60 $\mu$l of 0.053 M cobaltous perchlorate and 50 $\mu$l of 30% hydrogen peroxide. The pH was adjusted to 8.0 by means of 0.1 N sodium hydroxide and the mixture was allowed to stand at 25°C for 3 hours. The reaction was stopped by gelfiltration on Sephadex® G-25 (Eluent: water) whereupon unreacted insulin was separated by ion-exchange chromatography on Q-Sepharose® Fast Flow. A gradient elution system was employed (Buffer A: 20 mM HEPES pH 8.0 and Buffer B: 20 mM HEPES pH 8.0 plus 1 M sodium perchlorate).

Unreacted insulin was eluted at ca. 15% B buffer whereas the product was eluted at ca. 30% B buffer. The $(Inh)_6(Co(III))_2$-containing fractions were pooled, desalted on Sephadex® G-25 (Eluent: water) and concentrated by ultrafiltration.

Example 3

Preparation of $(Inh)_6(Co(III))_2$ with ammonia coordinated

55 mg of metal-free human insulin was dissolved in 5 ml of 2.5% ammonia (adjusted to pH 8.0 by means of perchloric acid) whereupon 60 $\mu$l of 0.053 M cobaltous perchlorate and 50 $\mu$l of 30% hydrogen peroxide was added. The mixture was allowed to stand for 16 hours at 25°C after which the reaction was stopped by gelfiltration on Sephadex® G-25 (Eluent: water). Unreacted insulin was separated and the product obtained as described in Example 2.

Example 4

Preparation of $(Inh)_6(Co(III))_2$ with TRIS coordinated

55 mg of metal-free human insulin was dissolved in 5 ml of 50 mM TRIS (adjusted to pH 8.0 by means of perchloric acid) whereupon 60 $\mu$l of 0.053 M cobaltous perchlorate and 50 $\mu$l of 30% hydrogen peroxide was added. The further procedure was as described in Example 3.

Example 5

Preparation of $(Inh)_6(Co(III))_2$ with imidazole coordinated

To a solution of 55 mg of metal-free human insulin in 5 ml of water was added 60 $\mu$l of 0.053 M cobaltous perchlorate, 14 mg (0.2 mmole) of imidazole and 50 $\mu$l of 30% hydrogen peroxide. The further procedure was as described in Example 3.

## Example 6

Preparation of $(Inh)_6(Co(III))_2$ with histidine coordinated

To a solution of 55 mg of metal-free human insulin in 5 ml of water was added 60 μl of 0.053 M cobaltous perchlorate and 50 μl of 30% hydrogen peroxide. The pH was adjusted to 8.0 by means of 0.1 N sodium hydroxide and the mixture was allowed to stand at 25°C for 3 hours. Hydrogen peroxide and excess cobaltous perchlorate was then removed from the reaction mixture by gelfiltration on Sephadex® G-25 (Eluent: water). To the resulting solution containing the $(Inh)_6(Co(III))_2$ was added 80 mg of histidine (0,52 mmole), pH was adjusted to 8.0 by means of sodium hydroxide and the mixture was stirred for 24 hours at 25°C. Unreacted insulin and excess histidine was separated by ion-exchange chromatography as described in Example 2.

## Example 7

Preparation of $(Inh)_6(Co(III))_2$ with ethylenediamine coordinated

To a solution of 55 mg of metal-free human insulin in 5 ml of water was added 60 μl of 0.053 M cobaltous perchlorate and 50 μl of 30% hydrogen peroxide. The pH was adjusted to 8.0 by means of 0.1 N sodium hydroxide and the mixture was allowed to stand at 25°C for 3 hours. Excess of hydrogen peroxide and cobaltous perchlorate was then removed from the reaction mixture by gelfiltration on Sephadex® G-25 (Eluent: water). To the resulting solution containing the $(Inh)_6(Co(III))_2$ was added 40 μl of ethylenediamine, pH was adjusted to 8.0 by means of perchloric acid and the mixture was allowed to stand for 24 hours at 25°C. Unreacted insulin and excess ethylenediamine was separated by ion-exchange chromatography as described in example 2.

## Example 8

Preparation of $(Inh)_6(Co(III))_2$ with chloride ions coordinated

55 mg of metal-free human insulin was dissolved in 5 ml of 1 M sodium chloride, the pH of the solution was adjusted to 8.0 by means of sodium hydroxide whereupon 60 μl of 0.053 M cobaltous perchlorate and 50 μl of 30% hydrogen peroxide was added. The further procedure was as described in Example 3.

## Example 9

Preparation of an injectable solution

10.000 IU of a compound according to the general formula I are dissolved in 80 ml of sterile water. 0.9 g of sodium chloride and optionally a preservative is added and the pH is adjusted to 7.4. Sterile water is then added to a final volume of 100 ml. This solution contains 100 IU/ml of insulin.

Characterization of the products

The products obtained in Example 2-8 were characterized by their UV and CD spectra and by their retention behaviour in a high performance size exclusion chromatographic (HPSEC) system.
The results are given in the tables below

**TABLE 1**

HPSEC retention times of the products obtained in Example 2-8 (Column: Protein Pak 125 (250x10 mm). Eluent: 2.5 M acetic acid, 4 mM L-arginine, 4% acetonitrile. Flow rate: 1.0 ml/min. Detection UV absorption 254 nm).

5

| Compound | Retention time (min) |
| --- | --- |
| Human insulin (monomer) | 9.26 |
| Co(II) insulin | 9.25 |
| Human insulin (covalent dimer) | 8.31 |
| Example 2 | 7.37 |
| Example 3 | 7.60 |
| Example 4 | 7.49 |
| Example 5 | 7.38 |
| Example 6 | 7.41 |
| Example 7 | 7.39 |
| Example 8 | 7.43 |

**TABLE 2**

UV-absorption maxima (400-700 nm) with corresponding estimated molar extinction coefficients (E) and CD absorption bands with corresponding estimated values of delta epsilon (dE) for the products obtained in Example 2-8. Values of E and dE refers to the cobolt concentration (calculated assuming 2.0 Co(III) per hexamer insulin) and is given in $M^{-1}cm^{1}$.

| Compound | $UV_{max}$ | | $CD_{min}$ | | $CD_{max1}$ | | $CD_{max2}$ | |
| --- | --- | --- | --- | --- | --- | --- | --- | --- |
| | nm | E | nm | dE | nm | dE | nm | dE |
| Example 2 | 522 | 155 | 442 | 0.0465 | 529 | 0.575 | sh581 | 0.434 |
| Example 3 | 508 | 260 | 447 | 0.0401 | 530 | 0.528 | 583 | 0.435 |
| Example 4 | 507 | 244 | 443 | 0.0315 | 529 | 0.534 | sh581 | 0.401 |
| Example 5 | 523 | 165 | 485 | -0.0295 | 551 | 0.418 | absent | |
| Example 6 | 525 | 182 | 447 | 0.0560 | 529 | 0.638 | sh585 | 0.389 |
| Example 7 | 511 | 306 | 444 | 0.0545 | 527 | 0.667 | sh585 | 0.387 |
| Example 8 | 520 | 181 | 448 | 0.0756 | 531 | 0.504 | 584 | 0.435 |

sh refers to a shoulder in the CD spectrum

## IN VIVO TESTING OF Co(III)-INSULIN

Co(III)-insulin produced as described in Example 1 was injected subcutaneously in different doses to rabbits as a 100 IU/ml preparation. Blood glucose was monitored at intervals for 7 hours after the injection and compared to results obtained after injection of human insulin Actrapid®. Six rabbits were used at each dosage level. The results are listed in Table 3.

## TABLE 3

Effect of cobolt(III)-insulin according to Example 1 on the blood glucose level after s.c. injection to rabbits in different doses. Data given are the average of experiments in six rabbits.

### Blood glucose (% of level at 0 hours)

| Time (hours) | | 1 | 2 | 4 | 6 | 7 |
|---|---|---|---|---|---|---|
| Preparation | Dose | | | | | |
| Actrapid® | 2 IU | 53.0 | 52.9 | 90.9 | 94.6 | 98.9 |
| Co(III)-insulin | 4 IU | 68.7 | 57.5 | 63.8 | 76.1 | 81.2 |
| Co(III)-insulin | 6 IU | 60.7 | 57.8 | 58.1 | 57.6 | 68.6 |
| Co(III)-insulin | 8 IU | 63.6 | 53.0 | 52.5 | 53.5 | 64.3 |
| Co(III)-insulin | 10 IU | 62.5 | 53.6 | 51.7 | 48.9 | 55.6 |

All rabbits survived the experiment and even at the highest dose of the preparation according to the invention they showed no sign of hypoglycaemia. 10 IU of Actrapid® would certainly have caused hypoglycaemia in the rabbits and even 8 IU or perhaps 6 IU might also have this effect.

## Claims

1. A protracted insulin composition characterized in that it comprises an insulin complex of the general formula I

$$(Ins)_6(CO(III))_2(L)_m(M)_n \qquad (I)$$

wherein Ins is a naturally occuring insulin or an insulin analogue which has insulin effect in humans, L is a nitrogen containing ligand which can bond to Co(III) via a nitrogen atom, M is a ligand comprising no nitrogen atoms such as a water molecule or an anion which can bond to Co(III) with the proviso that if n is 2 or higher, M may designate different species at the same time, and n is zero or an integer between 1 and 6, and m is 6-n, when L is a monodentate ligand or (6 - n)/2, when L is a bidentate ligand and a suitable carrier for use as a medicament.

2. A pharmaceutical composition according to Claim 1 wherein m is zero.

3. A pharmaceutical composition according to Claim 1 or 2 wherein Ins is human insulin.

4. A pharmaceutical composition according to Claim 1 or 2 wherein Ins is porcine insulin.

5. A pharmaceutical composition according to Claim 1 or 2 wherein Ins is an insulin analogue.

6. A pharmaceutical composition according to any of the preceding claims, characterized in that it contains between 10 IU and 2000 IU per ml of a compound of the general formula I, preferably between 10 IU and 1000 IU, more preferred between 10 IU and 300 IU per ml of a compound of the general formula I.

7. Use of an insulin complex of the general formula I of Claim 1 for the preparation of a medicament for treating diabetes mellitus.

**Patentansprüche**

1. Insulinzusammensetzung mit Langzeitwirkung, dadurch gekennzeichnet, daß sie einen Insulinkomplex der allgemeinen Formel I

$$(Ins)_6(Co(III))_2(L)_m(M)_n \qquad (I)$$

wobei Ins ein natürlich auftretendes Insulin oder ein Insulinanalog ist, das in Menschen Insulinwirkung besitzt, L ein stickstoffhaltiger Ligand ist, der sich über ein Stickstoffatom an Co(III) binden kann, M ein keine Stickstoffatome umfassender Ligand ist, wie etwa ein Wassermolekül oder ein Anion, der sich an Co(III) binden kann, mit der Maßgabe, daß, falls n 2 oder mehr ist, M gleichzeitig unterschiedliche Spezies bezeichnen kann, und n 0 oder eine ganze Zahl zwischen 1 und 6 ist und m 6-n ist, wenn L ein einzähniger Ligand ist, oder (6-n)/2, wenn L ein zweizähniger Ligand ist, und einen geeigneten Trägerstoff zur Verwendung als ein Arzneimittel umfaßt.

2. Pharmazeutische Zusammensetzung nach Anspruch 1, wobei m 0 ist.

3. Pharmazeutische Zusammensetzung nach Anspruch 1 oder 2, wobei Ins Humaninsulin ist.

4. Pharmazeutische Zusammensetzung nach Anspruch 1 oder 2, wobei Ins Schweineinsulin ist.

5. Pharmazeutische Zusammensetzung nach Anspruch 1 oder 2, wobei Ins ein Insulinanalog ist.

6. Pharmazeutische Zusammensetzung nach einem der vorangehenden Ansprüche, dadurch gekennzeichnet, daß sie zwischen 10 IU und 2000 IU pro ml einer Verbindung der allgemeinen Formel I enthält, vorzugsweise zwischen 10 IU und 1000 IU, bevorzugter zwischen 10 IU und 300 IU pro ml einer Verbindung der allgemeinen Formel I.

7. Verwendung eines Insulinkomplexes der allgemeinen Formel I von Anspruch 1 für die Herstellung eines Arzneimittels zur Behandlung von Diabetes mellitus.

**Revendications**

1. Composition d'insuline à effet prolongé, caractérisée en ce qu'elle comprend un complexe d'insuline de formule générale I

$$(Ins)_6(CO(III))_2(L)_m(M)_n \qquad (I)$$

dans laquelle Ins est une insuline naturelle ou un analogue d'insuline ayant une activité d'insuline chez l'homme, L est un ligand contenant de l'azote qui peut se lier à Co(III) par l'intermédiaire d'un atome d'azote, M est un ligand ne contenant pas d'atome d'azote tel qu'une molécule d'eau ou un anion qui peut se lier à Co(III), sous réserve que, si n est supérieur ou égal à 2, M peut représenter différentes espèces en même temps, et n est zéro ou un entier compris entre 1 et 6, et m est 6-n quand L est un ligand monodenté ou (6-n)/2 quand L est un ligand bidenté, et un support convenable pour l'utilisation comme médicament.

2. Composition pharmaceutique selon la revendication 1, dans laquelle m est zéro.

3. Composition pharmaceutique selon la revendication 1 ou 2, dans laquelle Ins est l'insuline humaine.

4. Composition pharmaceutique selon la revendication 1 ou 2, dans laquelle Ins est l'insuline porcine.

5. Composition pharmaceutique selon la revendication 1 ou 2, dans laquelle Ins est un analogue d'insuline.

6. Composition pharmaceutique selon l'une quelconque des revendications précédentes, caractérisée en ce qu'elle contient entre 10 UI et 2000 UI par ml de composé de formule générale I, de préférence entre 10 UI et 1000 UI, de façon davantage préférée entre 10 UI et 300 UI par ml d'un composé de formule générale I.

7. Utilisation d'un complexe d'insuline de formule générale I selon la revendication 1 pour la préparation d'un médicament pour le traitement du diabète sucré.